# EUROPEAN PATENT SPECIFICATION

(11) **EP 0 457 413 B1**
(45) Date of publication and mention of the grant of the patent: **26.10.1994**
(21) Application number: 91201186.3
(22) Date of filing: 16.05.1991
(51) Int. Cl.: A61F 13/02

(54) **Wound dressing and method for the production thereof**
Wundverband und Verfahren zu seiner Herstellung
Pansement pour blessure et son procédé de fabrication

(30) Priority: 17.05.1990 NL 9001156
(43) Date of publication of application: 21.11.1991
(73) Proprietor: KONINKLIJKE UTERMÖHLEN N.V., NL-3526 KS Utrecht (NL)
(72) Inventor: Pijzel, Willem Ewout, NL-3632 AT Loenen A/D Vecht (NL)
(74) Representative: de Bruijn, Leendert C.

(56) References cited:
- EP-A- 0 206 697
- EP-A- 0 351 016
- FR-A- 2 044 554
- FR-A- 2 247 197
- US-A- 3 157 178
- US-A- 3 285 245

## Description

The invention relates to a wound dressing and a method for the production thereof.

The wound dressing according to the invention comprises:
A. a layer intended to be brought into contact with the wound, and
B. a cushion which is applied to that surface of layer (1) which does not come into contact with the wound, said cushion comprising:
   1. porous, fluid-permeable laminating film adjacent to layer (1), and
   2. fluid-absorbent material adjacent to layer (2).

A wound dressing of this type is described in EP-A-0 206 697. According to this publication a hemostatic adhesive bandage comprises an absorbant pad attached to an adhesive backing. Said pad is covered by a perforated film-type non-adherent wound release cover which is coated with a very thin coating of a high molecular weight polyethylene oxide. This polyethylene oxide coating may or may not have openings corresponding to those on the release cover. This means that this plastic film functions as a wound release cover surface so that when the bandage is removed the scab will not be disrupted and reinitiate bleeding.

The wound dressing according to the invention is intended in particular for the treatment of skin defects such as burns and lacerations as well as abrasions. It is known that so-called "artificial skin" can be used for the treatment of skin defects of this type. "Artificial skin" is in general "biocompatible" polymer material in the form of membranes. Membranes of this type have a pore structure such that, on the one hand, bacteria are kept outside the body and, on the other hand, an optimum passage of wound fluid is possible.

In principle, all types of artificial skin disclosed to date can be used as layer (1) of the dressing according to the invention. A number of these are discussed briefly below:
An artificial skin which is built up from two membranes adhering to one another is, for example, disclosed in Macromol. Chem.Rapid Communications 4. 675-680 (1983). The underlayer to be applied to the wound bed consists of a biodegradable polylactic acid/polyurethane mixture and contains pores having a diameter of 40-200 µm to promote tissue growth. After application, this underlayer is gradually hydrolysed and replaced by newly-formed tissue. The top layer, in contrast, consists of a non-biodegradable polyurethane and contains pores having a diameter of 0.5-1.0 µm. This top layer, which serves to protect the wound against a bacterial invasion and must ensure adequate transport of water vapour, is said to be pulled off the skin wound after healing.

In Burns 11, 274-280 an artificial skin based on a polyurethane membrane is described which is marketed under the name "omiderm". More particularly, Omiderm is built up from a polyurethane film to which hydrophilic monomers such as hydroxymethyl acrylate and/or acrylamide have been grafted.

Another artificial skin available commercially is Biobrane (Burns 7, (1979), 123-130). More particularly, this Biobrane artificial skin consists of a flexible nylon fabric with a protective silicone rubber membrane. Both layers are coated with a layer of hydrophilic collagen peptides. The top silicone rubber layer is mechanically punctured in a regular manner to enable drainage of wound fluid in this way.

The present invention relates to a wound dressing of the type as described in the preamble, which is characterized in that there is an elastomeric layer which is able to adhere to the wound and is permeable to wound fluid and which is provided with pores having a diameter of 20-200 µm, on the side which comes into contact with the wound, and in that the adhesion of layer (2) of the wound cushion to the layer (1) which is able to adhere to the wound is less than the adhesion of layer (1) to the wounded skin.

An essential difference between the bandage known from EP-A-0 206 697 is the use of the layer which comes into contact with the wound. According to said publication this is a very thin layer of polyethylene oxide which is able to form association compounds with certain blood proteins, such as prothrombin and fibrinogen, thus increasing the local concentration of the these proteins, which gives an increase in viscosity of the blood plasma which slows the flowing blood and, consequently, causes the hemostatic effect. However, according to the present invention it does not cause any chemical reaction. It attaches physically and not chemically to the wound, so that a barrier function is present.

In the case of the wound dressing according to the invention the artificial skin used is preferably an artificial skin for which exclusive rights are applied for in NL-A-8801741 or an artificial skin which is described in NL-A-90,00113. These patent applications correspond to EP-A-0,351,016 and EP-A-0,443,641, respectively. Preferably, layer (1) comprises a flat product which has a porosity of more than 75% and contains so-called macropores having a diameter in the range of 20-200 µm. In this product, pores having a diameter of 0.1-25 µm and in particular 1-20 µm are present in the walls of the macropores.

The wound dressing according to the invention contains, as layer (1), an elastomeric layer which is permeable to wound fluid and which is provided, on the side which does not come into contact with the wound, with micropores, that is to say pores which are smaller than the abovementioned macropores and which generally have a diameter of at most 0.7 µm and preferably 0.1-0.5 µm.

The thickness of layer (1) of the wound dressing according to the invention is not critical and in total can be, for example, 0.1-1 mm.

A particularly advantageous product is obtained if, in the wound dressing according to the invention, the layer (1) has a porosity of more than 75%, further pores having a diameter of 0.1-25 µm are present in the walls of the pores of layer (1), and that side of layer (1) which does not come into contact with the wound is provided with pores having a diameter of at most 0.7 µm and preferably 0.1-0.5 µm. Specifically, layer (1) has the following combination of desired properties, layer (1) being indicated as "artificial skin":
1) the artificial skin instantaneously and easily adheres firmly to the wound surface and remains permanently attached there to until the wound healing process is complete;
2) the artificial skin can be draped flexibly over the wound surface, air bubbles being displaced through the material;
3) the artificial skin immediately alleviates the pain arising from a wound;
4) the artificial skin has haemostatic properties;
5) the artificial skin reduces the production of wound fluid;
6) the artificial skin allows ample passage of wound fluid and thus prevents the formation of blisters under the material. By combination with a wound fluid-absorbent material lying thereon, the passage of wound fluid through the artificial skin can be greatly increased, for example from 1800 g/m².h to 4400 g/m².h (measured under a hydrostatic pressure of 2 cm H₂O);
7) the artificial skin prevents the penetration of bacteria but permits the passage of antimicrobial agents applied;
8) the artificial skin promotes the bactericidal characteristics of the wound surface and can even be applied to contaminated wounds;
9) the artificial skin becomes transparent after application to the wound surface, so that the course of the healing process can be monitored in a simple manner;
10) the artificial skin reduces the contraction which occurs with wounds;
11) the artificial skin improves the recovery rate of the epidermis and the quality of the healed epidermis;
12) the artificial skin gives no allergic reactions;
13) the artificial skin is relatively easy to use and can be stored for a long time; and
14) the artificial skin is simple to sterilise (for example by gas sterilisation or gamma sterilisation).

The wound dressing according to the invention comprises, on the one hand, a porous, fluid-permeable layer (2) adjacent to layer (1). A porous polyethene or polyurethane sheet can, for example, be used as layer (2). In fact, the fluid-permeable laminating sheet (or laminating film) serves to stick the absorbent wound cushion (3) to the artificial skin (layer (1)) in an easily detachable manner.

When the wound dressing according to the invention is applied to a skin defect, the layer (1) will adhere to the wound by means of capillary action. The system comprising layers (2) and (3) remains present on the layer (1) for a certain period in order (particularly at the start of the healing process), to allow exuded wound fluid to pass through (layer (2)) and to be absorbed (layer 3). After the desired period, however, the layers (2) and (3) can be removed from the layer (1), the pulling force exerted for the removal of the layers (2) and (3) being so low that the adhesion of the artificial skin layer (1) to the wound is not disturbed. Disturbance of this type would, after all, adversely affect the healing process.

The invention therefore relates to a wound dressing in which the materials are chosen such that the adhesion of the layer (2) of wound cushion to the layer which is able to adhere to the wound is less than the adhesion of layer (1) to the wounded skin.

The absorbent material (3), on the side which faces away from the wound, is preferably coated or impregnated with a hydrophobic material. Hydrophobic materials are generally "non-wovens" which are permeable to air and are provided with a water-repellent finish. The non-wovens are usually manufactured from polypropene, polyethene or viscose. Breathing, water-repellent films can also be used.

The nature of the fluid-absorbent material (3) which is used in the wound dressing according to the invention is not critical. Preferably, however, viscose is used which is processed in the form of fibres which are needled to give a non-woven. It is, however, equally possible to use cotton fibres or absorbent materials which are termed "absorbers" in the technology of wound dressings and hygienic dressings.

It is obvious that the wound dressing can be provided with further means for fixing to the skin. In this context, for example, fixing plasters may be considered.

The wound dressing according to the invention is preferably produced by laminating the layers (1) and (2)/(3). For example, it is possible, with the aid of an installation known per se, to soften (melt) the layer (2) of the system of layers (2) and (3) by supplying heat. It is also possible to use a conventional plastic as adhesive, which plastic can be applied either in the form of a film or as loose grains.

After the adhesive has been softened or melted, the layer (1) is brought into contact with the layer (2) by means of a pressure roller (calendering).

Another production method for the production of the wound dressing according to the invention comprises attaching the elastomeric layer (1) to the layer (2) using water-soluble glue. In this case it suffices to provide only a small surface area of the layers to be combined with glue.

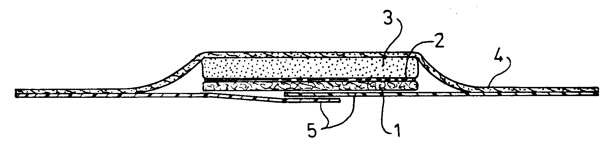

One embodiment of the wound dressing according to the invention is illustrated in the drawing, in which a cross-section is shown. In this drawing 1 indicates the skin-replacing membrane, that is to say the elastomeric layer of the wound dressing according to the invention. The second part is formed by a porous, fluid-permeable laminating film layer (2) and a fluid-absorbent wound cushion layer (3). A hydrophobic layer can be present on wound cushion layer (3) in order to prevent the wound cushion absorbing fluid on the side which faces away from the wound. A sticking plaster for attaching to the skin is indicated by 4. In the state in which it is available commercially, the adhesive layer of plaster 4 is provided with cover means 5, for example consisting of paper or plastic treated with a stripping agent (silicones), which can easily be detached from the adhesive layer of plaster 4.

## Claims

1. Wound dressing, comprising:
A. a layer (1) intended to be brought into contact with the wound, and
B. a cushion which is applied to that surface which does not come into contact with the wound, said cushion comprising:
1. porous, fluid-permeable laminating film (2) adjacent to layer (1), and
2. fluid-absorbent material (3) adjacent to layer (2),
characterised in that there is
an elastomeric layer (1) which is able to adhere to the wound and is permeable to wound fluid and which is provided with pores having a diameter of 20-200 µm, on the side which comes into contact with the wound, and in that the adhesion of layer (2) of the wound cushion to the layer (1) which is able to adhere to the wound is less than the adhesion of layer (1) to the wounded skin.

2. Wound dressing according to Claim 1, in which the layer (1) has a porosity of more than 75%, further pores having a diameter of 0.1-25 µm are present in the walls of the pores of layer (1), and that side of layer (1) which does not come into contact with the wound is provided with pores having a diameter of at most 0.7 µm and preferably 0.1-0.5 µm.

3. Wound dressing according to Claim 1 or 2, in which the absorbent material (3), on the side which faces away from the wound, is coated or impregnated with a hydrophobic material.

4. Wound dressing according to Claims 1-3, in which the absorbent material (3) is viscose.

5. Wound dressing according to Claims 1-4, provided with further means (4) for fixing to the skin.

6. Method for the production of a wound dressing according to Claims 1-5, in which the elastomeric layer (1) adheres to the layer (2) of the wound cushion as a result of calendering.

7. Method for the production of a wound dressing according to Claims 1-6, in which the elastomeric layer (1) adheres to the layer (2) of the wound cushion with the aid of water-soluble glue.

## Patentansprüche

1. Wundverband mit:
A. einer Schicht (1), die bestimmt ist, mit der Wunde in Kontakt gebracht zu werden, und
B. einem Kissen, das auf der Seite angebracht wird, die mit der Wunde nicht in Kontakt kommt, wobei das Kissen versehen ist mit:
1. einem der Schicht (1) benachbarten, porösen, fluidpermeablen Laminierfilm (2), und
2. einem der Schicht (2) benachbarten fluidabsorbierenden Material (3),
dadurch gekennzeichnet, daß
auf der Seite, die mit der Wunde in Kontakt kommt, eine elastomere Schicht (1), die an der Wunde haften kann und die für Wundfluid permeabel ist und die mit Poren mit einem Durchmesser von 20-200 µm versehen ist, vorgesehen ist, und daß die Adhäsion der Schicht (2) des Wundkissens an der Schicht (1), die an der Wunde haften kann, kleiner als die Adhäsion der Schicht (1) an der verwundeten Haut ist.

2. Wundverband nach Anspruch 1, wobei die Schicht (1) eine Porosität von mehr als 75 % hat, wobei in den Wänden der Poren der Schicht (1) weitere Poren mit einem Durchmesser von 0,1-25 µm vorhanden sind, und wobei die Seite der Schicht (1), die nicht in Kontakt mit der Wunde kommt, mit Poren versehen ist, die einen Durchmesser von höchstens 0,7 µm und vorzugsweise von 0,1-0,5 µm haben.

3. Wundverband nach Anspruch 1 oder 2, wobei das absorbierende Material (3) auf der von der Wunde wegweisenden Seite mit einem hydrophoben Material überzogen oder imprägniert ist.

4. Wundverband nach einem der Ansprüche 1 bis 3, wobei das absorbierende Material (3) Viskose ist.

5. Wundverband nach einem der Ansprüche 1 bis 4, wobei dieser mit einer zusätzlichen Anordnung (4) zum Fixieren an der Haut versehen ist.

6. Verfahren für die Herstellung eines Wundverbands nach einem der Ansprüche 1 bis 5, wobei die elastomere Schicht (1) infolge von Kalandrieren an der Schicht (2) des Wundkissens haftet.

7. Verfahren für die Herstellung eines Wundverbands nach einem der Ansprüche 1 bis 6, wobei die elastomere Schicht (1) mit Hilfe eines wasserlöslichen Leims an der Schicht (2) des Wundkissens haftet.

## Revendications

1. Pansement pour blessure, comprenant :
A. une couche (1) destinée à être mise en contact avec la blessure, et
B. un tampon qui est appliqué sur la surface qui ne vient pas au contact de la blessure, ledit tampon comprenant :
1. un film de laminage (2) poreux, perméable aux fluides, adjacent à la couche (1), et
2. un matériau (3) absorbant les fluides, adjacent à la couche (2),
caractérisé en ce qu'il comporte
une couche élastomère (1) qui est capable d'adhérer à la blessure et est perméable aux fluides de la blessure et qui est pourvue de pores présentant un diamètre allant de 20 à 200 µm, sur le côté qui vient en contact avec la blessure, et en ce que l'adhésion de la couche (2) du tampon pour blessure à la couche (1) qui est capable d'adhérer à la blessure est inférieure à l'adhésion de la couche (1) à la peau blessée.

2. Pansement pour blessure selon la revendication 1, dans lequel la couche (1) présente une porosité supérieure à 75 %, d'autres pores présentant un diamètre allant de 0,1 à 25 µm sont présents dans les parois des pores de la couche (1) et le côté de la couche (1) qui ne vient pas au contact de la blessure est pourvu de pores présentant un diamètre d'au maximum 0,7 µm et allant de préférence de 0,1 à 0,5 µm.

3. Pansement pour blessure selon la revendication 1 ou 2, dans lequel le matériau absorbant (3) est recouvert ou imprégné avec une matière hydrophobe sur le côté qui est tourné à l'opposé de la blessure.

4. Pansement pour blessure selon l'une des revendication 1 à 3, dans lequel la matière absorbante (3) est du viscose.

5. Pansement pour blessure selon l'une des revendications 1 à 4, pourvu d'un moyen (4) supplémentaire de fixation à la peau.

6. Procédé de fabrication d'un pansement pour blessure selon l'une des revendications 1 à 5, dans lequel la couche élastomère (4) adhère à la couche (2) du tampon pour blessure, suite à un laminage.

7. Procédé de fabrication d'un pansement pour blessure selon l'une des revendications 1 à 6, dans lequel la couche élastomère (1) adhère à la couche (2) du tampon pour blessure à l'aide d'une colle soluble dans l'eau.
